# EUROPÄISCHE PATENTSCHRIFT

(11) **EP 3 641 697 B1**
(45) Veröffentlichungstag und Bekanntmachung des Hinweises auf die Patenterteilung: **06.04.2022**
(21) Anmeldenummer: 18749290.5
(22) Anmeldetag: 12.06.2018
(51) Int. Cl.: A61D 7/00, A61M 15/00, A61M 16/06, A61M 11/00, A61D 7/04

(54) **INHALATOR FÜR PFERDE**
INHALER FOR HORSES
INHALATEUR POUR CHEVAUX

(30) Priorität: 20.06.2017 DE 102017005834; 06.09.2017 DE 102017008459
(43) Veröffentlichungstag der Anmeldung: 29.04.2020
(73) Patentinhaber: MARX, Stefan, 66701 Beckingen (DE)
(72) Erfinder: MARX, Stefan, 66701 Beckingen (DE)
(86) Internationale Anmeldenummer: PCT/DE2018/000186
(87) Internationale Veröffentlichungsnummer: WO 2018/233737

(56) Entgegenhaltungen:
- EP-A1- 2 298 230
- WO-A1-93/13730
- DE-A1- 10 260 636
- DE-A1- 10 322 505
- DE-A1-102005 033 808
- DE-A1-102007 059 570
- US-A1- 2002 056 456
- US-A1- 2014 261 398

## Beschreibung

Die Erfindung betrifft einen Inhalator für Pferde mit einer kappenartigen, über Maul- und Nüstern eines Pferdes stülpbaren Maske und innerhalb der Maske angeordnete Einrichtungen zur Erzeugung eines unter der Maske durch das Pferd zu inhalierenden Aerosols, wobei die Einrichtungen zur Erzeugung des Aerosols eine Vernebelungseinheit mit einer aufladbaren Batterie, einem Ultraschallzerstäuber und einem Vorratsbehälter für zu zerstäubende Flüssigkeit aufweisen. Wenn bei Pferden ein Wirkstoff mit besonders hohem Wirkungsgrad möglichst direkt in die Lunge oder über die Lunge in die Blutbahn übergehen soll, hat es sich seit langem bewährt, dem Pferd eine Maske über Nüstern und Maul zu stülpen, in welche der Wirkstoff als Aerosol, also als ein Gemisch aus Luft und feinen Tröpfchen des flüssigen Wirkstoffs, eingesprüht wird. Dieses Prinzip der Wirkstoffverabreichung bietet gegenüber der Einspritzung in die Nüstern den Vorteil eines erheblich geringeren Bedarfs an Wirkstoff bei schnellerer Einwirkung.

Es sind z.B. im Reitsport eingesetzte Inhalatoren für Pferde der eingangs erwähnten Art bekannt, deren Einrichtungen zur Erzeugung des Aerosols auf der Außenseite der kappenartigen Maske angeordnet sind. Gebildetes Aerosol wird durch eine Öffnung in der Maskenwand ins Innere der Maske geleitet.

Inhalatoren mit im Inneren der Maske angeordneten Einrichtungen zur Aerosolerzeugung gehen aus DE 103 22 505 A1, DE 10 2005 033 808 A1, WO 93/13730 A1, DE 102 60 636 A1, US 2014/0261398 A1, EP 2 298 230 A1 und US 2002/0056456 hervor. Die Einrichtungen zur Aerosolerzeugung dieser bekannten Inhalatoren sind jeweils innerhalb eines Maskenraumes angeordnet, der von dem Maskenraum, in welchen der Kopf des Pferdes mit Maul und Nüstern unmittelbar eintaucht, abgesetzt ist. Ein Inhalator mit den Merkmalen des Oberbegriffs des Anspruchs 1 geht aus

DE 10 2007 059 570 A1 hervor. Die kappenartige Maske dieses bekannten Inhalators weist in einem den Nüstern gegenüberliegenden Seitenwandabschnitt eine seitliche Ausbuchtung auf, in welcher die Einrichtungen zur Aerosolbildung untergebracht sind. Am Kappenboden befindet sich ein Rückschlagventil. An ihrer offenen Seite weist die Maske eine den Kopf des Pferdes umschlingende Ringdichtung auf.

Der Erfindung liegt die Aufgabe zugrunde, einen neuen Inhalator der eingangs genannten Art zu schaffen, der gegenüber diesem Stand der Technik konstruktiv vereinfacht ist.

Der diese Aufgabe lösende Inhalator nach der Erfindung ist in Anspruch 1 definiert.

Erfindungsgemäß sind die Einrichtungen zur Erzeugung des Aerosols innerhalb der kappenartigen Maske an dem Maskenboden geschützt angeordnet. Beschädigungen dieser Einrichtungen durch Stöße z.B. bei Kopfbewegungen des Pferdes sind vermieden. Erfindungsgemäß sind die Einrichtungen zur Erzeugung des Aerosols innenseitig an den Maskenboden koppelbar und von diesem abkoppelbar.

Vorzugsweise weist die Maske Öffnungen für die Durchströmung mit Atemluft des Pferdes auf. Während Atemluft andererseits durch die Hauptöffnung der übergestülpten Maske zwischen dem Kopf des Pferdes und dem Öffnungsrand eintreten kann, erleichtern diese Öffnungen die Ein- und Ausströmung von Atemluft.

In weiterer vorteilhafter Ausgestaltung der Erfindung ist die topfartige Maske mit Verbindungseinrichtungen zur Anbringung am Kopf des Pferdes, insbesondere zum Einhängen an einem Kopfgeschirr, versehen. Vorteilhaft kann das Pferd mit aufgesetzter Maske dann während der Inhalation in Schritt- oder Trabbewegung sein, was die Durchdringung der Atemwege und Lunge mit Aerosol wesentlich begünstigt und das Aerosol bis in die Alveolen der Lunge gelangen lässt.

Die Einrichtungen zur Erzeugung eines Aerosols umfassen ferner zweckmäßig ein die Verneblereinheit aufnehmendes Gehäuse. Reinigung und Wartung der Maske sowie der Aerosolerzeugungseinrichtungen sind so wesentlich erleichtert.

Insbesondere weist das Gehäuse eine Öffnung auf, durch die ein eine Aerosolaustrittsöffnung bildender Rohransatz der Verneblereinheit hindurchragt. Vorteilhaft lässt sich das Gehäuse leicht durch Ausspülen mit Wasser reinigen und die Maske damit kurzfristig zur Weiterverwendung für ein anderes Pferd herrichten. Die Verneblereinheit weist vorzugsweise eine Steckerbuchse für ein Lade- oder Spannungsversorgungskabel auf. Vorteilhaft kann bei entladener Batterie der Verneblereinheit der Inhalator durch eine fremde Spannungsquelle mit Betriebsspannung versorgt werden.

In einem weiteren (nicht beanspruchten) Inhalator umfasst die Maske einen topfförmigen Maskenteil, einen Topfboden und eine den Topfboden überwölbende Endkappe. Vorzugsweise trennt der Topfboden vollständig den Innenraum der Endkappe vom Innenraum des topfförmigen Maskenteils ab.

Der Topfboden kann fest mit der Endkappe verbunden sein. Vorzugsweise ist er oben unlösbar mit dem Maskenteil verbunden.

Zweckmäßig ist die Endkappe lösbar mit dem Maskenteil verbunden, insbesondere über einer Schraub-Dreh-Verbindung, vorzugsweise über einen Bajonettverschluss. Die nicht beanspruchten Einrichtungen zur Erzeugung des Aerosols können sowohl innerhalb des Maskenteils als auch in der Endkappe angeordnete Einrichtungsteile umfassen. Vorzugsweise sind innerhalb des Maskenteils ein Vorratsbehälter und mindestens eine Zerstäubereinheit und innerhalb der Endkappe eine Elektronikbaugruppe und eine aufladbare Batterie angeordnet, wobei die Elektronikbaugruppe sowohl zum Betrieb die Zerstäubereinheit als auch zwecks Ladung die Batterie ansteuert. Insbesondere sind der Vorratsbehälter und die mindestens eine Zerstäubereinheit in einer Baugruppe zusammengefasst, die in Richtung zu dem Topfboden in den Maskenteil einschiebbar und mit der Topfwand verbindbar ist. Zweckmäßig ist die Zerstäubereinheit lösbar mit dieser Baugruppe verbunden.

Die Baugruppe kann ein gewölbtes Wandelement umfassen, das unter Einschluss des Vorratsbehälters und der mindestens einen Zerstäubereinheit zwischen dem Wandelement und der Topfwand bündig in die Topfwand übergeht. Die innerhalb des topfförmigen Maskenteils angeordnete Baugruppe steht daher vorteilhaft von der Topfwand nicht nach innen.

Zweckmäßig lassen sich im Zuge der Verbindung der Endkappe mit dem Maskenteil automatisch elektrische Verbindungen zwischen der Elektronikbaugruppe und der mindestens einen Zerstäubereinheit oder/und zu der Solarzelle herstellen.

In weiterer Ausgestaltung der Erfindung umfassen die Einrichtungen zur Erzeugung des Aerosols zwei räumlich jeweils einem der beiden Nüster des Pferdes zugeordnete Zerstäubungseinheiten. Entsprechend weist das Wandelement den Nüstern des Pferdes gegenüberliegende Austrittsöffnungen für das Aerosol auf.

In einer Ausführungsform der Erfindung kann das Maskenteil auf seiner Außenseite eine Solarzelle aufweisen. Vorzugsweise ist die an der Maske seitlich angebrachte Solarzelle in die Topfwand eingesenkt und schließt bündig mit der Außenseite der Topfwand ab. Beschädigungen werden so vermieden.

Die Offenbarung ist nachfolgend anhand von Beispielen und der beiliegenden, sich auf diese Beispiele beziehenden Zeichnungen weiter erläutert. Es zeigen:
- Fig. 1: einen Inhalator nach der Erfindung in einer Seitenansicht,
- Fig. 2: ein Detail des Inhalators von Fig. 1,
- Fig. 3: eine Teilansicht des Inhalators von Fig. 1 mit den, erfindungsgemäß, innerhalb des Inhalators ange-ordneten Einrichtungen zur Bildung eines Aerosols,
- Fig. 4: die Einrichtungen zur Bildung des Aerosols im geöffneten Zustand eines durch die Einrichtungen umfassten Gehäuses,
- Fig. 5: eine in den Einrichtungen zur Erzeugung des Aerosols verwendete Verneblereinheit,
- Fig. 6: die Inhalatorkappe von Fig. 1 mit der außenseitig und somit nicht erfindungsgermäßen Verneblereinheit,

- Fig. 7: ein Lade- oder/und Spannungsversorgungskabel für die Vemeblereinheit von Fig. 5,
- Fig. 8: eine weitere (nicht beanspruchte) Ausführungsform für einen Inhalator in zueinander senkrechten Seitenansichten,
- Fig. 9: eine Detaildarstellung des nicht erfindungsgemäßen Beispiels von Fig. 8 in Explosionsdarstellung,
- Fig. 10: das nicht erfindungsgemäße Beispiel von fig. 8 in drei zueinander senkrechten geschnittenen Teilansichten, und
- Fig. 11: ein weiteres, nicht erfindungsgemäßes, Beispiel eines Inhalators mit einer Solarzelle.

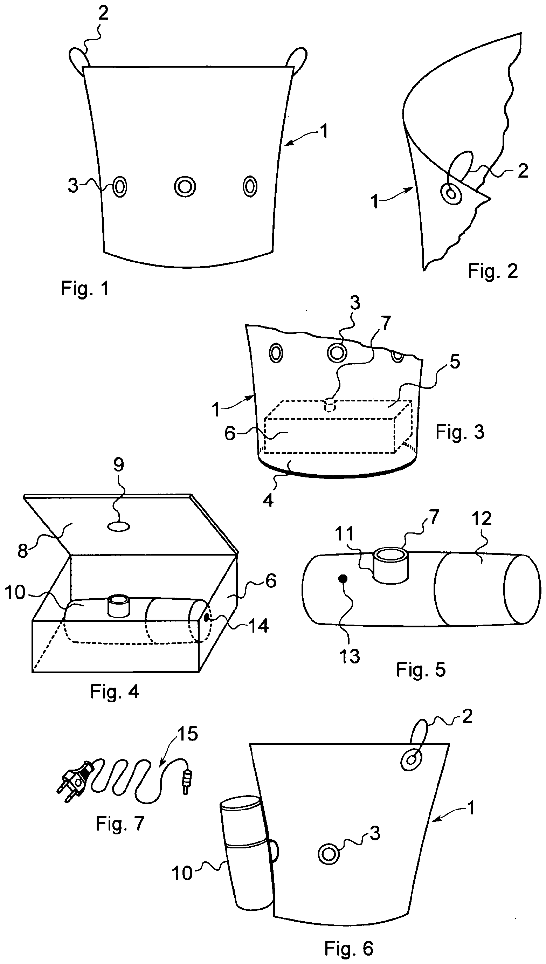

Ein Inhalator für Pferde umfasst eine kappen- oder haubenartige Maske 1, die über Maul und Nüstern eines Pferdes stülpbar und über Verbindungseinrichtungen 2, z.B. in Fig. 2 gezeigte Karabinerhaken, an einem Kopfgeschirr bzw. Halfter des Pferdes anbringbar ist. Wie Fig. 1 ferner erkennen lässt, weist die kappenartige Maske Öffnungen 3 auf, durch die Atemluft des Pferdes in die Maske ein- und aus der Maske ausströmen kann.

Wie aus Fig. 3 hervorgeht, sind auf einem Boden 4 der Maske 1 Einrichtungen 5 zur Erzeugung eines Aerosols angeordnet und ggf. auf dem Boden befestigt, z.B. durch eine einrastende Steckverbindung. Die Einrichtungen 5 weisen ein Gehäuse 6 und eine Aerosolaustrittsöffnung 7 auf. Abweichend von der gezeigten Quaderform könnte das Gehäuse 6 auch rund ausgebildet sein und insbesondere den Boden 4 vollständig bedecken.

Gemäß Fig. 4 weist das Gehäuse 6 einen Klappdeckel 8 mit einer Öffnung 9 auf und in dem Gehäuse 6 ist eine Verneblereinheit 10 mit einem die Aerosolaustrittsöffnung 7 bildenden Rohransatz 11 untergebracht. Bei geschlossenem Klappdeckel 8 ragt der Rohransatz 11 aus der Öffnung 9 heraus. Als im Einzelnen nicht gezeigte Bestandteile umfasst die Verneblereinheit 10 eine aufladbare Batterie sowie einen Ultraschallzerstäuber, z.B. mit einem Piezoschwingungselement. Ein Vorratsraum 12 dient der Aufnahme zu zerstäubender Flüssigkeit, vorzugsweise einer Kochsalzlösung. Durch einen Schaltknopf 13 lässt sich die Verneblereinheit ein- und ausschalten und ggf. programmieren, z.B. die Zeitdauer der Aerosolbildung einstellen (Timerfunktion). Die Verneblereinheit 10 weist ferner eine Anschlussbuchse 14 zur Ladung oder Spannungsversorgung durch eine externe Spannungsquelle (z.B. Powerbank) über ein in Fig. 6 gezeigtes Versorgungskabel 15 (z.B. USB-Kabel) auf. Es versteht sich, dass das Gehäuse 6 und die Maske 1 den Zugang zu der Anschlussbuchse 14 ermöglichende Öffnungen (nicht gezeigt) aufweisen.

Wie aus Fig. 7 hervorgeht, kann die Verneblereinheit 10 alternativ zu der Anordnung innerhalb der Maske 1 auch auf der Außenseite der Maske (allerdings nicht erfindungsgemäß) angeordnet werden, wobei der Rohransatz 11 durch eine der Öffnungen 3 in der Maske 1 hindurchragt und durch das in die Öffnung 3 ragenden Rohransatz 11 eine Steckverbindung hergestellt ist.

Die Öffnungen 3 sind an der Maske 1 zweckmäßig in einer solchen Position gebildet, dass die Erzeugung von Aerosol durch die Einrichtungen 5 innerhalb der Maske 1 durch die Öffnungen 3 hindurch sichtbar und kontrollierbar ist.

Die Verneblereinheit 10 weist vorzugsweise eine auf ihren Betrieb hinweisende Blinkanzeige auf.

Es versteht sich, dass die Maske 1 auch als Maulkorb verwendbar ist.

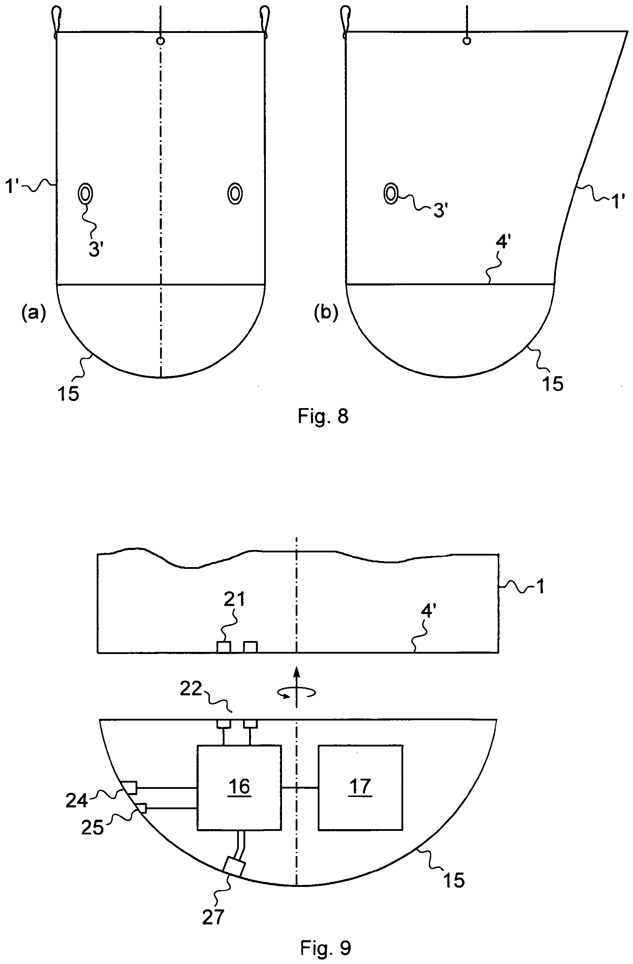

Ein anhand der Fig. 8 bis 11 beschriebenes, nicht erfindungsgemäßes, Beispiel für einen Inhalator ist zweiteilig mit einem topfförmigen Maskenteil 1' und einer Endkappe 15 ausgebildet. Die sich an einen Boden 4' des topfartigen Maskenteils 1' anschließende Endkappe 15 ist über einen im Einzelnen nicht dargestellten Bajonettverschluss mit dem topfförmigen Maskenteil 1' verbindbar. In der Endkappe 15 sind eine Elektronikbaugruppe 16 und eine aufladebare Batterie 17 untergebracht, wie dies in Fig. 9 schematisch dargestellt ist.

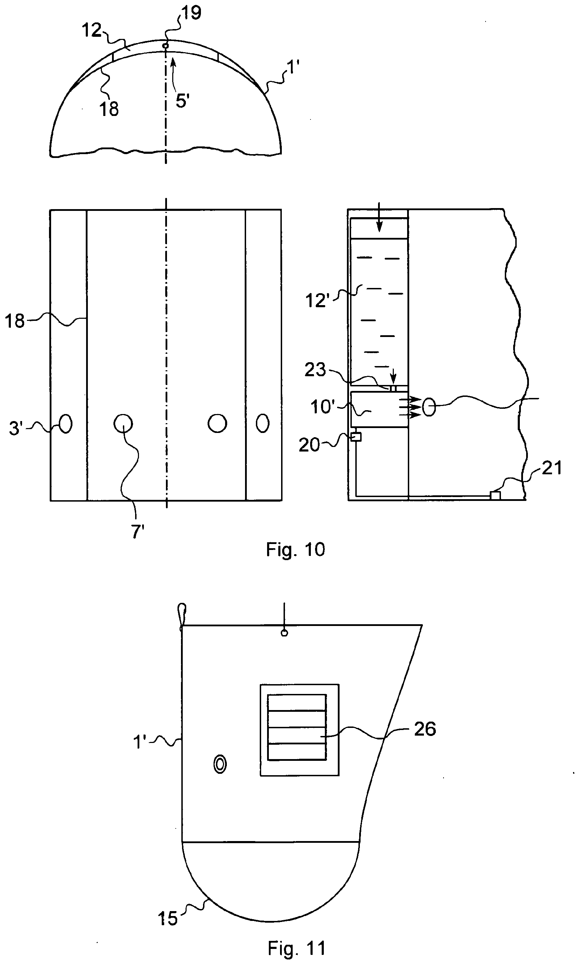

Der topfförmige Maskenteil 1' ist an die Kopfform des Pferdes angepasst und weitet sich von dem kreisrunden Boden 4 zum offenen Ende hin zu einer im Querschnitt etwa elliptischen Form auf. Innerhalb des topfförmigen Maskenteils 1' befindet sich eine Baugruppe 5' zur Erzeugung eines Aerosols. Wie Fig. 10 erkennen lässt, umfasst diese Baugruppe einen Vorratsbehälter 12' für eine zu vernebelnde Flüssigkeit sowie zwei Piezomembranen umfassende Zerstäubereinheiten 10. Sowohl der Vorratsbehälter als auch die Zerstäubereinheiten 10' sind mit einem gewölbten Wandelement 18 verbunden, in dem zwei, jeweils einer der Zerstäubereinheiten 10' zugeordnete Aerosolaustrittsöffnungen 7' gebildet sind.

Sowohl das Wandelement 18 als auch der Vorratsbehälter 12' ist aus durchsichtigem Material hergestellt, so dass jederzeit der Füllstand im Vorratsraum kontrolliert werden kann.

Die Baugruppe 5' zur Aerosolbildung lässt sich vom offenen Ende her bequem in den topfförmigen Maskenteil 1' einführen. In dem gezeigten Beispiel sind innenseitig an der Topfwand und am Vorratsbehälter 12' Schienenelemente (nicht gezeigt) angebracht, durch die die Baugruppe 5' geführt und formschlüssig an der Topfwand des topfförmigen Maskenteils 1' gehalten ist.

Zur Vorbereitung des Inhalatorbetriebs wird die Baugruppe 5' dem Maskenteil 1' entnommen und nach Kontrolle des Flüssigkeitsstandes ggf. zu vernebelnde Flüssigkeit in den Vorratsraum durch eine Öffnung 19 hindurch eingefüllt.

In dem in den Maskenteil 1' eingeschobenen Zustand geht das Wandelement 18 bündig in die Topfwand des Maskenteils 1' über. Mit dem Einschieben in die obengenannten Schienenelemente werden Kontakte 20 geschlossen. Diese Kontakte 20 stehen in Verbindung mit weiteren Kontakten 21, die bei der Verbindung der Endkappe 15 mit dem topfartigen Maskenteil 1' automatisch mit Kontakten 22 an der Endkappe 15 in Berührung kommen. Damit ist eine elektrische Verbindung zwischen der Elektronikbaugruppe 16 und den Zerstäubereinheiten 10' hergestellt. Bei Betätigung eines an der Endkappe 15 vorgesehenen Hauptschalters 27 werden die Zerstäubereinheiten 10' aktiv und Flüssigkeit, die über eine Verbindung 23 aus dem Vorratsbehälter 12' in die Zerstäubereinheiten 10' gelangt, wird durch die Öffnungen 7' hindurch in den Innenraum des Maskenteils 1' eingebracht. Die Öffnungen 7' sind entsprechend den Nüstern des Pferdes angeordnet, so dass Aerosol auf kürzestem Wege von den Zerstäubungseinheiten 10' in die Nüstern des Pferdes gelangen kann. Öffnungen 3' in der Topfwand des topfförmigen Maskenteils 1' sind am Umfang nur geringfügig versetzt zu den Öffnungen 7' angeordnet, so dass sie ebenfalls nahe bei den Nüstern angeordnet sind und das Atmen des Pferdes dadurch erleichtert ist.

An der Endkappe 15 befindet sich eine Anzeige 24 zur Kontrolle des Ladezustands der Batterie sowie eine Anschlussbuchse 25 zur Batterieladung.

Entsprechend einem in Fig. 11 gezeigten, nicht erfindungsgemäßen, Beispiels kann die Batterieladung zumindest teilweise über eine Solarzelle 26 erfolgen, die in die Topfwand des Maskenteils 1' bündig eingesenkt ist. Durch die Einsenkung sind Beschädigungen der Solarzelle weitgehend vermieden.

### Bezugszeichen:

- 1: - Maske
- 1': - Maskenteil
- 2: - Verbindungseinrichtungen
- 3,3': - Öffnungen
- 4: - Boden
- 4': - Topfboden
- 5: - Einrichtungen zur Erzeugung eines Aerosols
- 5': - Baugruppe zur Erzeugung eines Aerosols
- 6: - Gehäuse
- 7,7': - Aerosolaustrittsöffnungen
- 8: - Klappdeckel
- 9: - Öffnung
- 10: - Verneblereinheit
- 10': - Zerstäubereinheit
- 11: - Rohransatz
- 12: - Vorratsraum
- 12': - Vorratsbehälter
- 13: - Schaltknopf
- 14: - Anschlussbuchse
- 15: - Endkappe
- 16: - Elektronikbaugruppe
- 17: - Batterien
- 18: - Wandelement
- 19: - Öffnung
- 20: - Kontakte
- 21: - Kontakte
- 22: - Kontakte
- 23: - Verbindung
- 24: - Anzeige
- 25: - Anschlussbuchse
- 26: - Solarzelle
- 27: - Hauptschalter

## Patentansprüche

1. Inhalator für Pferde mit einer kappenartigen, über Maul und Nüstern eines Pferdes stülpbaren Maske (1) und innerhalb der Maske angeordneten Einrichtungen (5) zur Erzeugung eines unter der Maske (1) durch das Pferd zu inhalierenden Aerosols,
wobei die Einrichtungen (5) zur Erzeugung des Aerosols eine Verneblereinheit (10) mit einer aufladbaren Batterie, einem Ultraschallzerstäuber und einem Vorratsbehälter (12) für zu zerstäubende Flüssigkeit aufweisen, **dadurch gekennzeichnet,**
**dass** die Einrichtungen (5) zur Erzeugung des Aerosols innenseitig an einem der offenen Kappenseite gegenüberliegenden Boden (4) der Maske (1) koppelbar und von diesem Boden (4) abkoppelbar sind.

2. Inhalator nach Anspruch 1,
**dadurch gekennzeichnet,**
**dass** die Maske (1) Öffnungen (3) für die Durchströmung mit Atemluft des Pferdes aufweist.

3. Inhalator nach Anspruch 1 oder 2,
**dadurch gekennzeichnet,**
**dass** die Maske (1) mit Verbindungseinrichtungen (2) zur Anbringung am Kopf des Pferdes, insbesondere zum Einhängen an einem Kopfgeschirr, versehen ist.

4. Inhalator nach einem der Ansprüche 1 bis 3,
**dadurch gekennzeichnet,**
**dass** die Einrichtungen (5) zur Erzeugung des Aerosols ein die Verneblereinheit (10) aufnehmendes Gehäuse (6) aufweisen.

5. Inhalator nach Anspruch 4,
**dadurch gekennzeichnet,**
**dass** das Gehäuse (6) eine Öffnung (9) aufweist, durch die ein eine Areosolaustrittsöffnung (7) bildender Rohransatz (11) der Verneblereinheit (10) hindurchragt.

6. Inhalator nach einem der Ansprüche 1 bis 5,
**dadurch gekennzeichnet,**
**dass** die Verneblereinheit (10) eine Steckerbuchse (14) zur Verbindung mit einem Lade- oder Spannungsversorgungskabel (15) aufweist.

## Claims

1. Inhaler for horses, comprising a cap-like mask (1) which can be pulled over the mouth and nostrils of a horse, and, arranged inside the mask, devices (5) for generating an aerosol for the horse to inhale under the mask (1),
the devices (5) for generating the aerosol having a nebulizer unit (10) with a rechargeable battery, an ultrasonic atomizer and a storage container (12) for liquid that is to be atomized,
**characterized in that** the devices (5) for generating the aerosol can be coupled to the inner side of a bottom (4) of the mask (1) opposite the open cap side and can be uncoupled from this bottom (4).

2. Inhaler according to Claim 1, **characterized in that** the mask (1) has openings (3) allowing respiratory air of the horse to flow through.

3. Inhaler according to Claim 1 or 2, **characterized in that** the mask (1) is provided with connecting devices (2) for arranging it on the head of the horse, in particular for attaching it to a head harness.

4. Inhaler according to one of Claims 1 to 3, **characterized in that** the devices (5) for generating the aerosol have a housing (6) that receives the nebulizer unit (10).

5. Inhaler according to Claim 4, **characterized in that** the housing (6) has an opening (9) through which a tubular nozzle (11) of the nebulizer unit (10) projects, said tubular nozzle (11) forming an aerosol outlet opening (7).

6. Inhaler according to one of Claims 1 to 5, **characterized in that** the nebulizer unit (10) has a plug socket (14) for connection to a charging or power supply cable (15).

## Revendications

1. Inhalateur pour chevaux, présentant un masque (1) de type calotte pouvant être enfilé sur la bouche et les narines d'un cheval et des dispositif (5) agencés à l'intérieur du masque pour générer un aérosol à inhaler par le cheval sous le masque (1), les dispositifs (5) pour générer l'aérosol présentant une unité de nébulisation (10) pourvue d'une batterie rechargeable, d'un pulvérisateur à ultrasons et d'un réservoir (12) pour le liquide à pulvériser, **caractérisé en ce que** les dispositifs (5) pour générer l'aérosol peuvent être accouplés à l'intérieur à un fond (4) du masque (1) situé à l'opposé du côté ouvert de la calotte et désaccouplés de ce fond (4).

2. Inhalateur selon la revendication 1, **caractérisé en ce que** le masque (1) présente des ouvertures (3) pour l'écoulement de l'air expiré du cheval.

3. Inhalateur selon la revendication 1 ou 2, **caractérisé en ce que** le masque (1) est pourvu de dispositifs de raccordement (2) pour la fixation à la tête du cheval, en particulier pour la suspension à un licol.

4. Inhalateur selon l'une quelconque des revendications 1 à 3, **caractérisé en ce que** les dispositifs (5) pour générer l'aérosol présentent un boîtier (6) recevant l'unité de nébulisation (10).

5. Inhalateur selon la revendication 4, **caractérisé en ce que** le boîtier (6) présente une ouverture (9) à travers laquelle passe une tubulure (11) formant l'ouverture de sortie de l'aérosol (7) de l'unité de nébulisation (10).

6. Inhalateur selon l'une quelconque des revendications 1 à 5, **caractérisé en ce que** l'unité de nébulisation (10) présente un connecteur femelle (14) pour la connexion à un câble de charge ou d'alimentation en tension (15).
